# EUROPEAN PATENT APPLICATION

(11) **EP 1 955 670 A1**
(43) Date of publication of application: **13.08.2008**
(21) Application number: 08002061.3
(22) Date of filing: 04.02.2008
(51) Int. Cl.: A61C 8/00, A61B 17/86

(54) **Dental implant with constant thread crest width**

(30) Priority: 07.02.2007 US 900037 P
(71) Applicant: Biomet 3I, Inc., Palm Beach Gardens, FL 33410 (US)
(72) Inventor: Robb, Tait T., Stewart, FL 34997 (US)
(74) Representative: Grünecker, Kinkeldey, Stockmair & Schwanhäusser Anwaltssozietät

(57) **Abstract**

An implant for placement into bone and method for forming the same. The method provides an implant that has an elongated body that has a distal end and a proximal end. The method also provides a thread-cutting tool. The process engages the elongated body with the thread-cutting tool. The thread-cutting tool contacts the elongated body at the proximal end and removes material from the implant to form a thread and a tapered portion near the distal end of the elongated body. The thread-cutting tool forms the thread and a generally-cylindrical portion between the tapered portion and the proximal end of the elongated body. The thread has first crest width in the tapered region and a second crest width in the generally-cylindrical portion. The thread-cutting tool contacts the thread in the generally-cylindrical portion of the elongated body. The thread-cutting tool makes a second pass to reduce the second crest width.

## Description

### FIELD OF INVENTION

The invention relates to screw-type dental implants and, more particularly, to a dental implant with a thread crest having a reduced width.

### BACKGROUND OF THE INVENTION

Screw-type dental implants are widely used and have been known for a number of years. Two types of screw-type dental implants are self-tapping implants and non-self-tapping implants. Self-tapping implants are threaded into a pre-drilled bore in a jawbone without pre-tapping the bore, while non-self-tapping implants require that a pre-drilled borew also be tapped prior to the insertion of the non-self-tapping implant. In either type, the implant has a generally cylindrical main body that has at least one set of external screw threads on its outer surface. The external screw threads engage corresponding internal threads cut into the wall of the bore to provide initial stabilization of the implant in the bore.

Many current self-tapping screw-type dental implants contain a taper at a lower end of the implant, a tapping end of the self-tapping implants, so that a diameter of the implant at the lower end is smaller than a diameter of the rest if the implant. This taper is formed by pressing a thread-cutting tool further into rod stock material used to form the implant farther at the lower end than over the rest of the implant. As a result, the diameter is reduced over the portion of the implant where the thread-cutting tool is inserted more deeply. In addition to having a reduced diameter, the width of the crest of the thread in the tapered portion is also reduced, as the thread-cutting tool also has a tapered shape. Therefore, as the thread-cutting tool is not pressed into the rod stock material as far over the non-tapered section of the implant, the width of the thread crest in the non-tapered section is also greater. As a result of thread crest being wider over the non-tapered section of the implant, those threads increase the pressure on the threads formed within the bone by the narrower thread crest of the self-tapping section, and cause more compression of the bone. Thus, a need exists for a self-tapping screw-type dental implant with a constant thread crest width.

### SUMMARY OF THE INVENTION

According to one embodiment an implant for placement into bone tissue having an exterior surface comprises an elongated body that has a tapered portion, a generally-cylindrical portion, and at least one external thread. The thread makes a plurality of turns about the elongated body. The thread has a first crest width in the tapered portion and a second crest width in the generally-cylindrical portion. The first crest width is generally similar to the second crest width.

According to one process, a method forms a thread on an implant for placement into bone tissue. The implant has an elongated body with a distal end and a proximal end. The method engages the elongated body with a thread-cutting tool at the distal end of the elongated body. The thread-cutting tool removes material from the implant to form a thread and a tapered portion near the distal end of the elongated body. The thread-cutting tool forms the thread and a generally-cylindrical portion between the tapered portion and the proximal end of the elongated body. The thread has first crest width in the tapered region and a second crest width in the generally-cylindrical portion. The thread-cutting tool reduces the second crest width.

According to another process, a method of forms a thread on an implant for placement into bone tissue. The method provides an implant that has an elongated body that has a distal end and a proximal end. A thread-cutting tool is provided. The thread-cutting tool engages the elongated body. The thread-cutting tool contacts the elongated body at the distal end and removes material from the implant to form a thread while gradually decreasing the depth of the thread-cutting tool to form a tapered portion near the distal end of the elongated body. The thread of the tapered portion has a first crest width. The thread-cutting tool moves axially along the elongated body of the implant at the decreased depth and removes material from the elongated body to form the thread and a generally-cylindrical portion of the elongated body. The thread of the generally-cylindrical portion has a second crest width. The generally-cylindrical portion is located between the tapered portion and the proximal end. After the thread-cutting tool moves axially along the elongated body, the thread-cutting toll is positioned on the thread of the generally-cylindrical portion of the elongated body. The thread-cutting tool makes at least one additional pass along the generally-cylindrical portion of the elongated body to remove additional material from the elongated body and reduce the second crest width to a third crest width.

### BRIEF DESCRIPTION OF THE DRAWINGS

FIG. 1 is a side view of a dental implant according to one embodiment;

FIGS. 2a-c are side views of the machining of the threads into a dental implant according to one process;

FIG. 3 is a detailed view of the Area 3 of FIG. 2b;

FIG. 4 is a detailed view of the Area 4 of FIG. 2c.

While the invention is susceptible to various modifications and alternative forms, specific embodiments thereof have been shown by way of example in the drawings and will herein be described in detail. It should be understood, however, that it is not intended to limit the invention to the particular forms disclosed but, on the contrary, the intention is to cover all modifications, equivalents, and alternatives falling within the spirit and scope of the invention as defined by the appended claims.

### DESCRIPTION OF ILLUSTRATIVE EMBODIMENTS

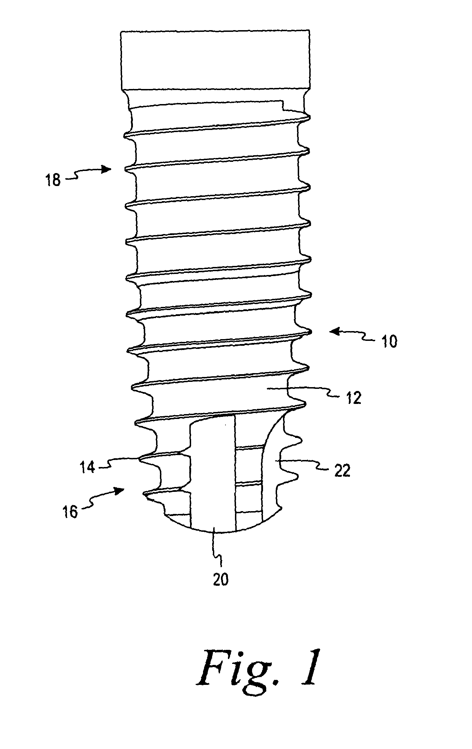

As shown in FIG. 1, a dental implant 10 is shown. The dental implant 10 has a main body 12 with external threads 14. The dental implant 10 has a tapered portion 16 and a generally-cylindrical portion 18. The dental implant 10 additionally features four axially elongated recesses or cut-outs, two of which 20, 22 are shown in FIG. 1, spaced symmetrically ninety degrees (90°) apart around a longitudinal axis of the implant 10. These recesses form cutting edges that are effective to self-tap the implant 10 into a pre-drilled bore in bone tissue.

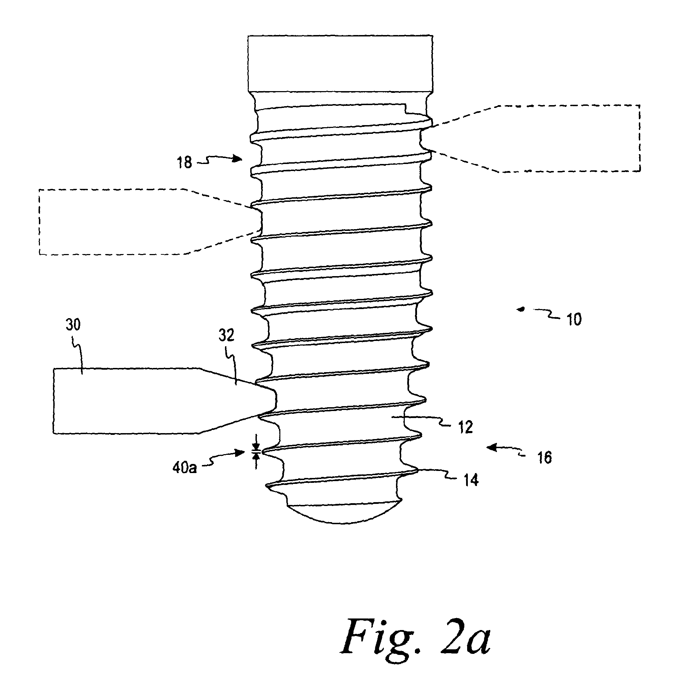

Turning now to FIGS. 2a-2c, a process of forming the threads 14 on the implant 10 is shown. The threads 14 may be cut with a thread cutting tool 30, that can be pushed into the main body 12 as the implant 10 is turned. As can be seen in FIGS. 2a-2c, the thread cutting tool 30 has a tapered end 32. The cutting tool 30 is shown in three positions in FIGS. 2a-2c to depict various stages of progression of the process used to form the threads 14. The cutting tool 30 is shown in broken lines in two of the three positions, and in solid line at one position, the solid line cutting tool 30 indicating the thread region currently being formed.

As can be seen by the position of the thread-cutting tool 30 in FIG. 2a, the tapered end 16 is formed by pressing the thread-cutting tool 30 further into the main body 12 of the implant 10 when the external threads 14 are cut. Thus, as the tapered end 32 of the cutting tool 30 is pushed farther into the main body 12 of the dental implant 10 to form the tapered end 16 of the implant 10, a first crest width 40a of the threads formed by the thread-cutting tool 30.

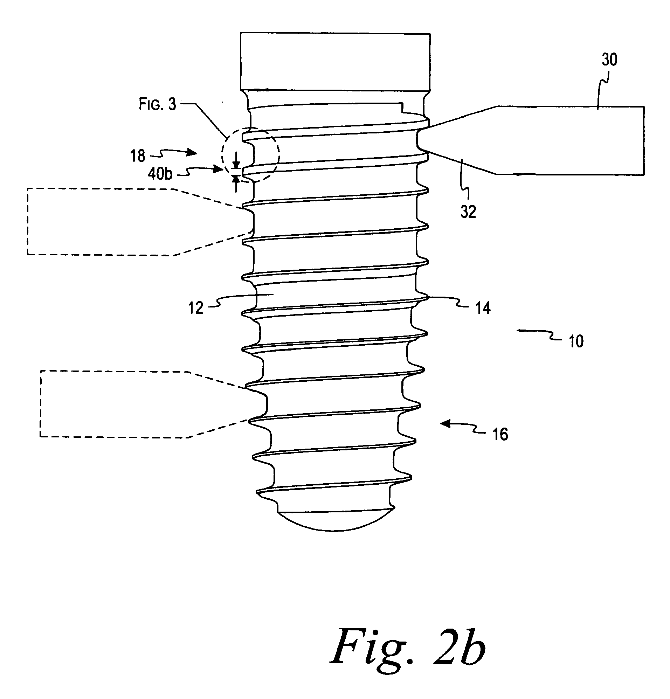

Turning next to FIG. 2b, the thread cutting tool 30 is shown creating external threads in the generally-cylindrical portion 18 of the implant 10. The cutting tool 30 is not pressed as far into the implant 10 in the generally-cylindrical portion 18 as in the tapered portion 16. Thus, a second crest width 40b is present after an initial pass of the thread-cutting tool 30 in the generally-cylindrical portion 18. The first crest width 40a in the tapered portion 16 is narrower than a second crest width 40b in the generally-cylindrical portion 18 of the implant 10 based on the taper of the thread-cutting tool 30 after a single pass of the thread-cutting tool 30.

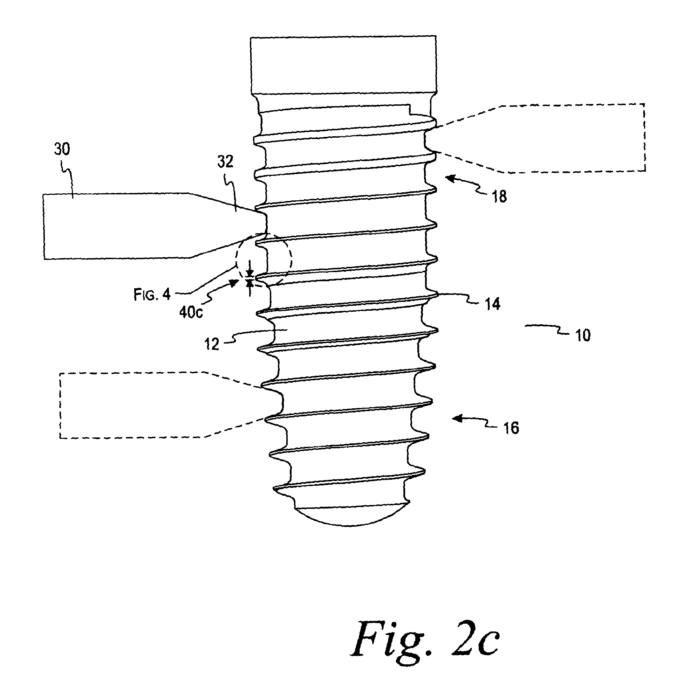

As shown in FIG 2c, in order to create a uniform crest width for all of the external threads 14, a second pass of the thread-cutting tool 30 is required within the generally-cylindrical portion 18 of the implant 10. The cutting tool 30 is moved relative to the implant 10 such that the additional material is removed from the main body 12 of the implant 10 by the cutting tool 30 during the second pass. This additional material is only removed from one side of the thread 14 of the generally-cylindrical portion 18.

The thread-cutting tool 30 that has been moved relative to the implant 10 removes additional material from the main body 12 of the implant 10 in the generally-cylindrical portion 18. By removing this additional material, a crest width 40c is formed in the generally-cylindrical portion 18, that has been reduced from the crest width 40b, that is generally identical the first crest width 40a of the tapered portion of the implant 10.

It is contemplated according to one process that the implant is maintained in a fixed location, and the thread-cutting tool is indexed relative to the fixed implant to form the reduced crest width. It is contemplated according to an alternative process that the implant is moved relative to a fixed location thread-cutting tool to form the reduced crest width.

Therefore, the removing of additional material by an additional pass of the thread-cutting tool 30 on the generally-cylindrical portion 18 of the implant 10 that results in a narrower crest width 40c in the generally-cylindrical section of the implant 10 that is generally similar to the crest width 40a of the tapered portion 16 of the implant. By having generally similar crest widths 40a, 40c over the length of the implant, the stress that the threads 14 place on bone tissue that an implant 10 is placed into is reduced. The stress placed on bone tissue is reduced as additional compressive forces are not applied to the bone tissue by the threads of the generally-cylindrical portion 18 of the implant as would be applied if the crest width of the thread in the generally-cylindrical portion was wider than the crest width of the thread in the tapered portion 16. In an implant with narrower crest widths in a tapered portion than a generally-cylindrical portion the tapered portion will compress the bone tissue a first amount, and then the generally-cylindrical portion will compress the bone tissue a second amount, greater than the first amount. The additional compression of the bone tissue by the wider crest width of the generally-cylindrical portion increases the stress on the bone tissue.

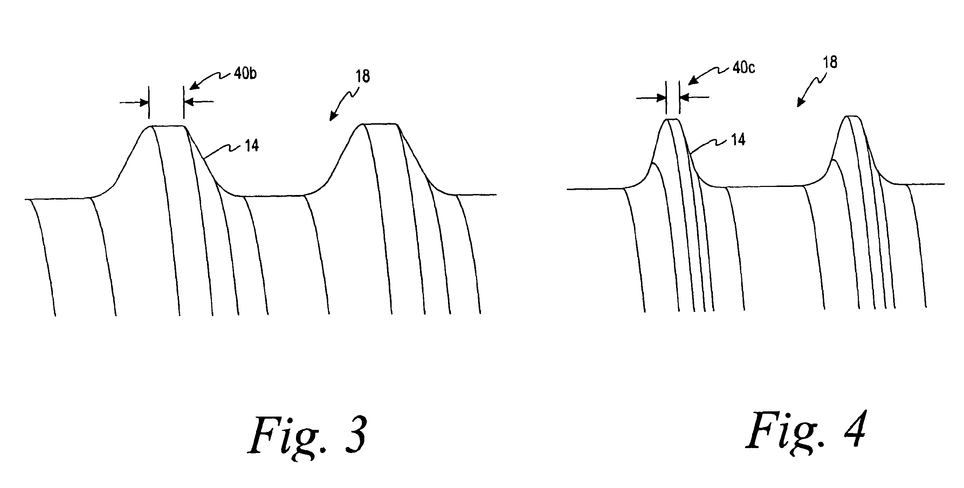

FIG. 3 depicts a detailed view of an area bound by circle 3 of FIG. 2b of the threads 14 of the generally-cylindrical portion 18 of the implant 10 after an initial pass of the thread-cutting tool 30 is shown. In the embodiment depicted, the crest width 40b is about 0.006 inches at a major diameter of the thread.

FIG. 4 shows a detailed view of an area bound by circle 4 of FIG. 2c of the threads 14 of the generally-cylindrical portion 18 of the implant 10 after at least one additional pass of the thread-cutting tool 30 is shown. The crest width 40c ranges from about 0.002 inches to about 0.004 inches.

In addition to reducing the mount of stress placed on bone the implant 10 is placed in, the reduced crest width 40c of the generally-cylindrical portion 18 of the implant 10 also reduces the torque required to drive the implant 10 into the bone. As the area of the thread interacting with the bone is reduced, less torques is thus required to place the implant 10.

It is further contemplated that the implant 10 may further undergo a surface treatment to enhance oseointegration, such as that described in U.S. Patent No. 6,969,474.

While particular embodiments and applications of the present invention have been illustrated and described, it is to be understood that the invention is not limited to the precise construction and compositions disclosed herein and that various modifications, changes, and variations may be apparent from the foregoing descriptions without departing from the spirit and scope of the invention as defined in the appended claims.

## Claims

1. An implant for placement into bone tissue having an exterior surface comprising:
an elongated body having a tapered portion and a generally-cylindrical portion; and
at least one external thread making a plurality of turns about the elongated body including the tapered portion and the generally-cylindrical portion of the elongated body, the thread having a first crest width in the tapered portion and a second crest width within the generally-cylindrical portion, wherein the first crest width is generally similar to the second crest width.

2. The implant of claim 1, wherein the elongated body has a distal end and a proximal end, the distal end for being submerged into bone tissue, the tapered portion being located near the distal end of the elongated body, the proximal end for being located near an exterior surface of the bone tissue, the generally-cylindrical portion being located between the tapered portion and the proximal end.

3. The implant of claim 2, wherein the thread within the generally-cylindrical portion has a relief area on a surface of the thread nearer the distal end.

4. The implant of claim 1, wherein the exterior surface on the elongated body and the external thread undergo a treatment to enhance oseointegration.

5. The implant of claim 1, wherein the second crest width is from about 0.002 inch to about 0.004 inch.

6. The implant of claim 1, wherein the tapered portion has a self-tapping feature.

7. The implant of claim 1, wherein the first crest width is generally similar to the second crest width at a major diameter of the thread.

8. A method of forming a thread on an implant for placement into bone tissue, the implant having an elongated body having a distal end and a proximal end comprising the acts of:
engaging the elongated body with a thread-cutting tool at the distal end of the elongated body;
removing material from the implant to form a thread and a tapered portion near the distal end of the elongated body, the thread having a first crest width in the tapered portion;
forming the thread and a generally-cylindrical portion between the tapered portion and the proximal end of the elongated body with the thread-cutting tool, the thread having a second crest width in the generally-cylindrical portion;
reducing the second crest width with the thread cutting tool.

9. The method of claim 8, wherein the thread-cutting tool has a taper.

10. The method of claim 8, wherein the act of contacting the thread in the generally-cylindrical portion removes material from a distal side of the thread.

11. The method of claim 8, wherein the thread-cutting tool is positioned farther from the distal end of the implant prior to the act of contacting the thread in the generally-cylindrical portion and the act of making a second pass with the thread-cutting tool.

12. The method of claim 11, wherein the thread-cutting tool is positioned farther from the distal end of the implant by moving the implant.

13. The method of claim 11, wherein the thread-cutting tool is positioned farther from the distal end of the implant by moving the thread-cutting tool.

14. The method of claim 8, wherein the act making a second pass with the thread-cutting tool reduces the second crest width from about 0.001 inches to about 0.005 inches.

15. The method of claim 14, wherein the act making a second pass with the thread-cutting tool reduces the second crest width about 0.003 inches.

16. A method of forming a thread on an implant for placement into bone tissue, the implant having an elongated body having a distal end and a proximal end, the method comprising the acts of:
engaging the elongated body with a thread-cutting tool, the thread-cutting tool contacting the elongated body at the distal end and removing material from the implant to form a thread while gradually decreasing the depth of the thread-cutting tool to form a tapered portion near the distal end of the elongated body, the thread of the tapered portion having a first crest width;
moving the thread-cutting tool axially along the elongated body of the implant at the decreased depth removing material from the elongated body forming the thread and a generally-cylindrical portion of the elongated body, the thread of the generally-cylindrical portion having a second crest width, the generally-cylindrical portion being located between the tapered portion and the proximal end;
after the act of moving the thread-cutting tool axially along the elongated body, positioning the thread-cutting on the thread of the generally-cylindrical portion of the elongated body; and
removing additional material from the elongated body reducing the second crest width to a third crest width by making at least one additional pass along the generally-cylindrical portion of the elongated body with the thread-cutting tool.

17. The method of claim 16, wherein the thread-cutting tool has a taper.

18. The method of claim 16, wherein the act of removing additional material removes material from a distal side of the thread.

19. The method of claim 16, wherein the act of positioning the thread-cutting tool positions the thread-cutting tool relative to the implant so that the thread-cutting tool is closer to the proximal end of the implant by moving the implant.

20. The method of claim 16, wherein the act of positioning the thread-cutting tool positions the thread-cutting tool relative to the implant so that the thread-cutting tool is closer to the proximal end of the implant by moving the thread-cutting tool.

21. The method of claim 16, wherein the act of removing additional material with the thread-cutting tool reduces the second crest width from about 0.001 inches to about 0.005 inches to form the third crest width.

22. The method of claim 16, wherein the act of removing additional material with the thread-cutting tool reduces the second crest width about 0.003 inches to form the third crest width.

23. The method of claim 16, wherein the act of removing additional material removes material from a surface of the thread of the generally-cylindrical portion closer to the distal end of the implant.
